# EUROPEAN PATENT SPECIFICATION

(11) **EP 0 513 832 B1**
(45) Date of publication and mention of the grant of the patent: **26.07.2000**
(21) Application number: 92108286.3
(22) Date of filing: 15.05.1992
(51) Int. Cl.: A61K 47/14

(54) **Use of dibutyl adipate and isopropyl myristate in topical and transdermal products**
Verwendung von Dibutyl-Adipat und Isopropyl-Myristat in topischen und transdermalen Produkten
Utilisation du dibutyle d'adipate et du myristate d'isopropyle dans des produits à usage topique au transdermal

(30) Priority: 17.05.1991 US 701944; 12.11.1991 US 790939
(43) Date of publication of application: 19.11.1992
(73) Proprietor: Bristol-Myers Squibb Company, New York, N.Y. 10154 (US)
(72) Inventor: Parab, Prakash V., Williamsville, NY 14221 (US)
(74) Representative: Kinzebach, Werner, Dr.

(56) References cited:
- EP-A- 0 423 929
- WO-A-85/03434
- WO-A-89/07951
- LU-A- 84 059
- BRITISH JOURNAL OF DERMATOLOGY vol. 118, no. 4, April 1988, LONDON pages 523 - 530; WATSON W.S. ET AL: 'THE EFFECT OF THE VEHICLE FORMULATION ON THE STRATUM CORNEUM PENETRATION CHARACTERISTICS OF CLOBETASOL 17-PROPIONATE IN VIVO'
- Clinical and Experimental Dermatology, vol. 10, 1985, pages 13-21
- Archiv für Dermatologische Forschung, vol. 248, 1974, pages 387 - 390

## Description

The invention relates to compositions for enhancement as well as the control of epidermal, dermal and transdermal penetration of various topically applied pharmacologically active agents utilizing dibutyl adipate or a mixture of dibutyl adipate and isopropyl myristate.

Intravenous infusion, intramuscular injection, buccal, oral, rectal routes, and so forth have been generally adopted as methods for administration of a wide variety of therapeutically active agents, such as antihypertensives, β-Blockers, antiarrhythmics, antianginal agents, vasodilators, antiemetics, antibacterial, antifungals, corticosteroids, retinoids, progestins, estrogens, androgens, analgesics and anti-inflammatories. When these therapeutically active agents are administered to humans or warm blooded animals by such routes, they enter the general circulation and produce the desired systemic Therapeutic effect. However, it is well-known that the aforementioned methods of administration have certain disadvantages. For example, the buccal and rectal administration often produce discomfort and aggravation to the patient. The intravenous and intramuscular routes are not only painful for the patient, but also must be performed by trained individuals. Oral administration, although generally acceptable by the patient, may have the disadvantages of poor absorption of the therapeutic agent from the gastrointestinal tract or degradation which may be caused by the acidic medium of the stomach, enzymes in gastrointestinal tract, interaction with ingested food or by rapid metabolism by the liver through which the drug must pass before it enters the systemic circulation.

Recognizing these disadvantages, many investigators have used transdermal route to deliver the therapeutically active agent into systemic circulation. Various therapeutic and cosmetic agents are used for the treatment of a number of skin conditions, for example, hydrocortisone for pruritus and erythema in atopic dermatitis, sulconazole nitrate for fungal infection of the skin, tretinoin for photoaging, and 5-fluorouracil for psoriasis and skin cancer. However, the skin of humans and other warm blooded animals provides an excellent barrier to the penetration of exogenous chemical substances. The outermost layer of the skin, the stratum corneum, offers maximum resistance to penetration, whereas the lower layers are relatively permeable. For the proper treatment of skin disorders or skin diseases, it is important that the pharmacologically active agent penetrates the stratum corneum and is made available at appropriate concentrations at the site of action which can be the stratum corneum, the viable epidermis, the epidermis-dermis junction, the dermis itself, or all the aforementioned layers of the skin depending upon the type of disorder or disease condition.

In certain skin conditions such as ichthyosis, callus or plaque psoriasis, the stratus corneum is thicker and thus can provide a significantly greater barrier to penetration of the drug, reducing its efficacy. However, in a few disease conditions, such as psoriasis, the stratum corneum is not intact and hence is more permeable than normal skin. As the disease or condition improves, there will be restructuring of the barrier and therefore, the resistance for the permeation of the therapeutically active ingredient will increase.

To achieve a consistent supply of therapeutic active ingredient at the site of action during the treatment of skin diseases, it has been found that the use of penetration enhancer is essential. Investigators have turned to various enhancing agents, for example, dimethyl sulfoxide, dimethylformamide, methyldecylsulfoxide (U.S. Patent No. 3,527,864), dimethylacetamide (U.S. Patent No. 3,472,931), and N-alkyl-2 pyrrollidone (U.S. Patent No. 3,696,516), for topical as well as systemic delivery of therapeutic active agents. However, the use of the aforementioned penetration enhancers is not without problems. For example the use of dimethylsulfoxide causes a foul taste and body odor, causes burning and erythema on the skin, reduces the relucency of the lens cortex and causes tissue necrosis in animals (Martindale, The Extra Pharmacopoeia, pages 1461-1463, Twenty-Seventh Edition, 1977). Dimethylformamide and dimethylacetamide also cause a sensation of burning and erythema on the skin. As a result, there exists a need for a novel agent to enhance the absorption through the skin of therapeutic agents which is devoid of the disadvantages and drawbacks that to date have characterized many of the prior art enhancing agents.

The present invention discloses such an agent as dibutyl adipate (DBA) and the combination of dibutyl adipate and isopropyl myristate (IPM).

Dibutyl adipate is marketed under the trade name CETIOL B® by Henkel Corporation as a low fat emollient for cosmetic products. It is disclosed as being a solvent for lipid soluble subatances and as possessing good spreading properties. It is also recommended for use in cosmetic products such as hair spray, hair fixatives and hand and body day creams.

Harding, Sohail and Busse, Clinical and Experimental Dermatology, Vol. 10, page 13-21, 1985, have used dibutyl adipate in the cream and ointment formulation of clobetasol propionate. However, their aim was to reduce percutaneous absorption of clobetasol propionate by incorporating in the base an oil, such as dibutyl adipate into which steroids would favorably partition and reduce percutaneous absorption.

Watson and Finlay, British Journal of Dermatology (1988), 118, pages 523-530, studied the stratum corneum penetration characteristics of a formulation consisting of tritium labeled clobetasol-17-propionate dissolved in low viscosity oil dibutyl adipate, wax and liquid paraffin matrix.

LU-A-84 059 describes compositions containing a steroid dissolved in an oily solvent, such as dibutyl adipate and/or isopropyl myristate. The concentration of the steroid in the solution is such that the degree of unsaturation is at least 3.

Altmeyer and Zaun, Arch. Derm. Forsch. 248, 387-390 (1974) examined solutions of respectively 0,1% triamcinolone acetonide, 0,1% triamcinolone acetonide cumaric acid ester, or a mixture of 0,05% of either substance in dibutyl adipate.

Isopropyl myristate is known as a penetration enhancer for topical preparations. However, the applicants are unaware of any reference where a synergestic effect was reported in skin penetration enhancement from the combination of DBA and IPM.

The present invention discloses that emollient solvents such as dibutyl adipate when used in an optimum amount, alone or in combination with isopropyl myristate, can enhance as well as control the epidermal, dermal and transdermal penetration of various topically applied preparations.

Various dermally effective pharmacological agents are known which can provide beneficial effects when applied topically to the skin to treat surface or subsurface diseases or for creating skin conditions which protect the skin from external factors. Other pharmacological agents are also known which can provide beneficial effects when absorbed into the systemic circulation. A composition of such systemically effective pharmacological agents in combination with dibutyl adipate or a mixture of dibutyl adipate and isopropyl myristate can greatly enhance the rate of penetration of agents through the skin and increase the amount absorbed into the systemic circulation. Thus, it is possible to have a systemic effect through topical application of said composition. The topical delivery of systemically effective pharmacologic agents can be of significant advantage in cases where drugs produce gastric problems, are not well absorbed when given orally, or are rapidly metabolized in the liver, e.g. the "first pass" effect. In such cases, the use of topical delivery can give a systemic response at lower dosage than required orally. Topical delivery also avoids the disadvantages present in the intravenous route of administration, which might otherwise be necessary to achieve effective blood levels at reasonable dosage amounts. Such dermatological agents can be made more beneficial by enhancing their penetration through the protective layer of the skin in accord the with present invention.

The present invention relates to the enhancement as well as the control of epidermal, dermal and transdermal penetration of various topically applied preparations. More specifically, the invention relates to composition and methods for enhancing and/or controlling epidermal, dermal and transdermal penetration of topically applied pharmacologically active agents by use of dibutyl adipate, or a combination of dibutyl adipate and isopropyl myristate.

An object of the present invention is to provide an agent for enhancing and/or controlling skin permeation of therapeutic agents.

It is also an object of the present invention to provide an agent which will enhance and/or control epidermal and dermal absorption of dermatological (that is, cosmetic or therapeutic) agents and enhance and/or control delivery through the skin and into the general circulation of systemically active therapeutic agents.

Another object of the invention is to provide an enhancing agent which is devoid of side effects.

Yet another object of the invention is to provide a composition utilizing such enhancing agents, which formulations are useful for topical application.

Still another objective of the invention is to provide a method for enhancing the skin penetration of therapeutic chemicals.

Other objectives, features and advantages of the invention will be apparent to those skilled in the art upon a study of this disclosure and appended claims.
Figure 1 shows the skin penetration of halobetasol propionate from FN9-28150-73 cream, FN9-28150-71 cream and Ultravate® Ointment.
Figure 2 shows the skin penetration of halobetasol propionate from FN9-28150-79 ointment, FN9-28150-80 ointment and Ultravate® Ointment.
Figure 3 shows the skin penetration of BMY 30047 from cream formulations FN9-28190-56, FN9-28190-51, FN9-28190-63 and 30047-C-03-A.
Figure 4 shows the results of rhino mouse assay of FN9-28190-20 in comparison to Retin A®.

It has been found that the dermal and transdermal penetration of a pharmacologically active compound can be substantially improved by incorporating the compound into a composition containing a dermal and transdermal effective amount of dibutyl adipate or a mixture of dibutyl adipate and isopropyl myristate.

This unexpected effect is quite useful in that it allows one to improve the dermal and transdermal delivery of pharmacologically active compounds from the composition, thereby allowing one to achieve the same level of efficacy with a lower overall concentration of the pharmacologically active compound in the composition.

Therefore, the present invention relates to a topical composition for enhancing skin penetration of a pharmacologically active agent comprising:
a) an effective amount of a pharmacologically active agent selected from an α- or β-hydroxycarboxylic acid, ketocarboxylic acid or ester, lactone or salt thereof;
b) dibutyl adipate in an amount such that the pharmacologically active agent is present in the composition in the form of a suspension, saturated solution or solution having a degree of unsaturation of not more than 1.5; and
c) a pharmaceutically acceptable non-toxic topical vehicle.

The present invention further relates to a topical composition for enhancing skin penetration of a pharmacologically active agent comprising:
a) an effective amount of a pharmacologically active agent selected from a steroid, retinoid and arotinoid or from an α- or β-hydroxycarboxylic acid, ketocarboxylic acid or ester, lactone or salt thereof;
b) a mixture of dibutyl adipate and isopropyl myristate in an amount such that the pharmacologically active agent is present in the composition in the form of a suspension, saturated solution or solution having a degree of unsaturation of not more than 1.5; and
C) a pharmaceutically acceptable non-toxic topical vehicle.

The compositions of the present invention may also contain other ingredients of the type commonly employed by those skilled in the art of compositions for topical application. These may include, for example, carriers, emollients, surfactants and/or an additional penetration enhancer.

A composition can be any suitable non-toxic or pharmaceutically acceptable topical carrier material or vehicle such as a solution, suspension, emulsion, lotion, cream, gel, ointment, liposomes, aerosol spray, polymeric gel, sol, a cataplasm, a plaster, a patch, film, or a tape preparation, which are well-known to those skilled in the art of topical pharmaceutical formulation.

The methods of this invention apply to topical compositions containing a wide variety of pharmacologically active agents including but not limited to:
steroids such as hydrocortisone, prednisolone, betamethasone and triamcinolone;
antiphoto-aging compounds such as retinoids, arotinoids; and
drugs to treat disturbed keratinization of skin such as α or β hydroxycarboxylic acid and related ketocarboxylic acid and ester, lactones or salt forms thereof.

Preferably the pharmacological agent is from 0.001% wt to 80% wt of total composition. However, the effective amount of a specific pharmacological agent will vary in accordance with parameters well understood by the physician or veterinarian. These parameters include the condition being treated, the age, weight and physical condition of the subject, and of course, the specific agent selected.

The optimum level of DBA or a mixture of DBA and IPM may be readily evaluated by a few simple tests such as those described herein.

In general, for a given label strength of a pharmacologically active agent, it is preferred to use a minimum amount of solvent DBA, or DBA and IPM to dissolve the drug completely and yet maintain favorable partition coefficient and diffusion through the stratum corneum of the skin.

The general rate of penetration is in order of optimal solubilized solution > dilute solution. It is also necessary to consider the contribution of other solvents on the solubility of the given strength of the pharmacologically active agent in the composition. Generally, the concentration of DBA is 0.1% wt % to 99 wt % and the concentration for IPM is from 0 wt % to 99.9 wt %. The more preferred concentration of DBA is from 0.1 wt % to 50 wt % and of IPM is from 1 wt % to 30 wt %. All percentages of composition components recited are, unless otherwise indicated, weight percentage (wt %) and are based upon the weight of the composition.

The present invention will be described with reference to the examples below but it is not deemed to be limited only to these examples.

### EXAMPLE 1

### In Vitro Skin Penetration Study

The following test method may be employed with human skin to determine epidermal or dermal or transdermal penetration of pharmacologically active compounds used in the practice of this invention. The procedure is also applicable to skin of other warm blooded animals.

### Skin Preparation

Normal excised human skin obtained from breast reduction or abdominal skin samples obtained from the Firefighters Skin Bank were used. The skin samples were stored in a freezer at -30°C until needed. Only skin that appeared normal was used. Historical evidence of chronic illness, skin disease or skin injury excluded use of skin samples in the study.

The skin obtained from the Firefighters Skin Bank was supplied as sterile, split-thickness skin with most of the underlying dermis already removed. The skin was thawed and rinsed in normal saline for about 30 minutes prior to use.

The skin obtained from breast reduction autopsy was full thickness skin. It was thawed at room temperature in normal saline followed by freezing on a microtome with carbon dioxide and sectioning to a layer around 200 micrometers thick. It was then stored in normal saline at 5°C until about 8 hours before use.

### Skin Penetration

The skin sections were mounted on flat-top Franz diffusion cells with a diffusional cross-section of 0.636 cm² or 1.8 cm². A 50 or 100 microliter sample of test formulation was placed on the stratum corneum surface of the skin in the donor compartment and the receptor compartment was filled with 4 to 8 ml of normal saline or 30% isopropanol in water. The selection of receptor fluid depended on the drug candidate whose penetration had to be evaluated. The main objective was to maintain sink conditions in the receptor compartment. The receptor fluid was well stirred throughout the experiment and the temperature was maintained by circulating water at 37°C through the water jacket of the diffusion cell. A 150 to 500 microliter sample was withdrawn from the receptor compartment at appropriate intervals and analyzed for drug content by HPLC or by scintillation counter for radioactive drug. The receptor fluid was replenished with normal saline after each withdrawal. All the receptor fluid and replenished fluid was thoroughly degassed before use.

### EXAMPLE 2

Halobetasol propionate (BMY 30056) is an ultra-potent steroid used topically for the treatment of dermatological conditions such as eczema and psoriasis. Halobetasol propionate is commercially available by Bristol-Myers Squibb as ULTRAVATE® Cream and ULTRAVATE® Ointment. The composition of these commercial products and also several experimental formulations are given in Table 1 and Table 2. ULTRAVATE® Cream is an oil in water emulsion containing isopropyl palmitate and isopropyl isostearate as emollients. The ULTRAVATE® Ointment is a nonaqusous emulsion of propylene glycol in a petrolatum base. The in vitro human skin penetration of these two compositions and experimental formulation FN8-1089-25 cream is given in Table 3 under Study I. It is observed that the ULTRAVATE® Cream has lower skin penetration than ULTRAVATE® Ointment. The presence of the emollients isopropyl palmitate and isopropyl isostearate, which are known to have a similar penetration enhancement property to isopropyl myristate, did not enhance the penetration of halobetasol propionate from ULTRAVATE® Cream in comparison to ULTRAVATE® Ointment. The increase in skin penetration of halobetasol propionate from ULTRAVATE® Ointment can be due to the presence of the penetration enhancer propylene glycol, as well as due to occlusion provided by petrolatum. The experimental cream FN8-1089-25 having 15 wt % dibutyl adipate (DBA) and 7 wt % propylene glycol (PG) showed poor penetration in comparison to ULTRAVATE® Ointment and was not superior to ULTRAVATE® Cream. The poor penetration of this experimental formulation is due to oversolubilisation of halobetasol propionate in the cream vehicle by dibutyl adipate and the resulting reduction of partitioning of halobetasol into the skin. These findings are similar to those reported by Harding et al. (Clinical and Experimental Dermatology, Vol. 10, page 13-21, 1985) who, when using 18 wt % DBA and 10 wt % PG in clobetasol propionate cream formulation, observed reduced penetration of clobetasol propionate. These findings confirm that DBA reduces percutaneous absorption of therapeutic agent when used in a concentration which oversolubilizes the drug in the formulation.

In a different human skin study, the in vitro skin penetration of halobetasol propionate from ULTRAVATE® Ointment, FN8-1114-16 cream and FN8-1114-13 cream were evaluated (Table 3, study II). ULTRAVATE® Ointment was used as a control in order to normalize the data, and to assist in the comparison of skin penetration of different formulations when evaluated for skin penetration in different human skin. The rank order of penetration at 72 hours is ULTRAVATE® Ointment > FN8-1114-16 cream > FN8-1114-13 cream.

ULTRAVATE® Cream contains isopropyl palmitate and isopropyl isostearate, which are reported to have skin penetration enhancement properties similar to isopropyl myristate (IPM). However, ULTRAVATE® Cream containing these fatty acid esters shows poor penetration in comparison to Ultravate® Ointment (Table 3, Study I). The cream formulation FN8-1114-16 containing 6 wt % IPM and 2 wt % DBA shows about a five-fold increase in skin penetration than ULTRAVATE® Cream (compare results from Study I and Study II, Table 3) and has about four-fifths of the penetration of ULTRAVATE® Ointment.

Cream formulation FN8-1114-13 containing 6 wt % IPM + 2 wt % DBA + 23 wt % PG showed about 3 times more penetration than ULTRAVATE® Cream but was not superior to ULTRAVATE® Ointment nor the cream FN8-114-16.

It can be concluded from Study I and Study II that (a) by using an optimum amounts of DBA and IPM which are just sufficient to dissolve halobetasol propionate in the cream, one can enhance the skin penetration of halobetasol propionate, (b) inclusion of PG, a known penetration enhancer, in the formulation containing optimum amounts of DBA and IPM does not improve the skin penetration of halobetasol propionate any further, and (c)a mixture of DBA and IPM in the new cream formulation showed better skin penetration of halobetasol propionate than Ultravate® Cream having a mixture of isopropyl isostearate and isopropyl palmitate. Hence, the combination of DBA and IPM has a superior skin penetration enhancing property than isopropyl isostearate or isopropyl palmitate or IPM.

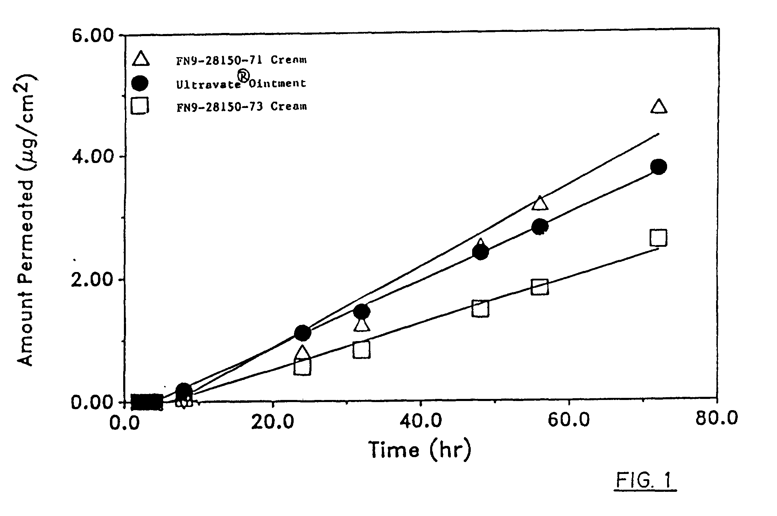

The in vitro skin penetration of ULTRAVATE® Ointment (control) and experimental cream FN9-28150-71 and FN9-28150-73 was evaluated in another experiment (Study III, Table 3, Fig. 1). The rank order of penetration at 72 hours is FN9-28150-71 cream ≥ ULTRAVATE® Ointment ≥ FN9-28150-73 cream.

The FN9-28150-71 cream contains 0.5 wt % DBA and 10 wt % IPM as solvents. This solvent mixture results in a saturated solution of 0.05 wt % of halobetasol propionate in the cream formulation. This cream showed about 7.5 times more enhanced skin penetration than ULTRAVATE® Cream and was slightly better than ULTRAVATE® Ointment (Compare study III with Study I in Table 3).

The FN9-28150-73 cream contains a 4 wt % DBA and 10 wt % IPM as solvents. The solvent concentration in this formulation is slightly in excess of what is necessary to dissolve the given strength of halobetasol propionate in the formula. Therefore, it gave comparatively less skin penetration than that of the formula having a saturated solution eg. FN9-28150-71 cream.

Generally petrolatum-based ointments, because of their occlusive nature, show superior skin penetration of pharmacologically active agents as compared to that of cream formulations. The present invention demonstrates that one can formulate cream formulations having appropriate proportions of IPM and DBA, to obtain skin penetration similar to or better than that of ointments.

Petrolatum-based ointments are also greasy in nature and in certain instances it may be desired to have cosmetically elegant (less greasy) topical products, such as creams, lotions, gels and solutions having penetrations of the pharmacologic active similar to that of an ointment. The present invention demonstrates such an achievement and therefore, the more cosmetically elegant formulation.

It is also clear from the above examples (Figure 1) that it is possible to control the rate of penetration of drug by just changing the proportion of DBA and IPM in the formula (compare example FN9-28150-71 with FN9-28150-73). This concept is very useful in dermal and transdermal products to control the rate of delivery of the drug to the site of action.

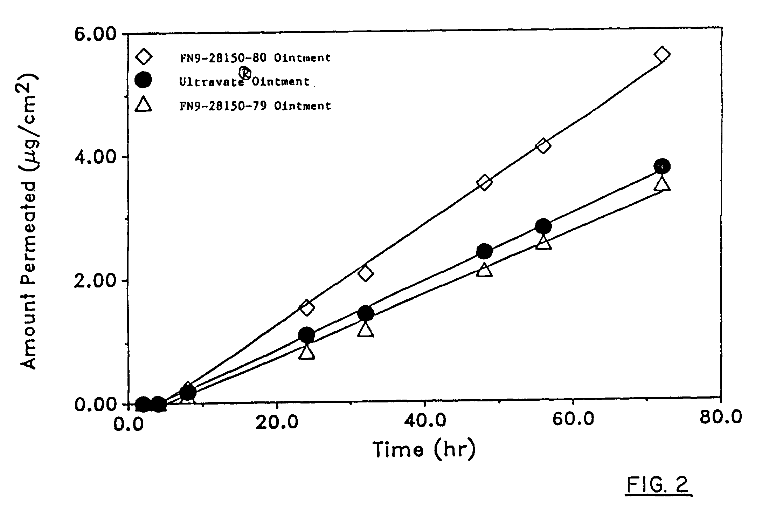

The in vitro skin penetration data of halobetasol propionate ointments FN9-28150-79 and FN9-28150-80, and commercial ULTRAVATE® Ointment as a control is shown in Fig. 2, and Study IV of Table 3. The rank order of penetration is FN9-28150-80 ointment > ULTRAVATE® Ointment ≥ FN9-28150-79 ointment.

The FN9-28150-79 ointment contains 2 wt % DBA and 6 wt % IPM in an optimum amount, just enough to dissolve halobetasol propionate in the ointment formulation. This new ointment gave penetration similar to that of the ULTRAVATE® Ointment containing PG as an enhancer.

The FN9-28150-80 ointment is similar to the FN9-28150-79 ointment but it contains ozokerite. This new ointment showed 1.5 times more penetration than ULTRAVATE® Ointment or the FN9-28150-79 ointment. This improved penetration is due to the use of an optimum mixture of IPM and DBA and may be due to the enhanced occlusion obtained by incorporating ozkerite in the ointment base.

It is a common practice to use PG as a penetration enhancer in dermal and transdermal preparations. PG, as discussed earlier, can be a sensitizer and irritant when applied topically. The side effects of PG are more pronounced in an occluded condition such as occurs in the application of an ointment or a transdermal patch (C. Huriez, P. Martin and M. Mennecier, L'allergie au propylene glycol, Rev. Franc. Allerg., Vol. 6: pages 200-205, 1966; M. Hannuksela, V. Pirila and O.P. Salo, Skin reactions to propylene glycol, Contact Dermatitis, Vol. 1: pages 112-116, 1975; S. Agren-Jonsson and B. Magnusson, Sensitization to propantheline bromide, trichlorocarbanilide and propylene glycol in an antiperspirant, Contact Dermatitis, Vol. 2: pages 79-80, 1976; and K. Motoyashi, S. Nozawa, M. Yosimura and K. Matsada, The safety of propylene glycol and other humectants, Cosmet. Toilet., Vol. 99: pages 83-91, October 1984.) The present invention demonstrates that by incorporating optimal amounts of dibutyl adipate and isopropyl myristate into a topical formulation, the formulation can have the benefits of enhanced skin penetration of pharmacologically active compounds without the use of propylene glycol or the possible associated irritation or sensitization.

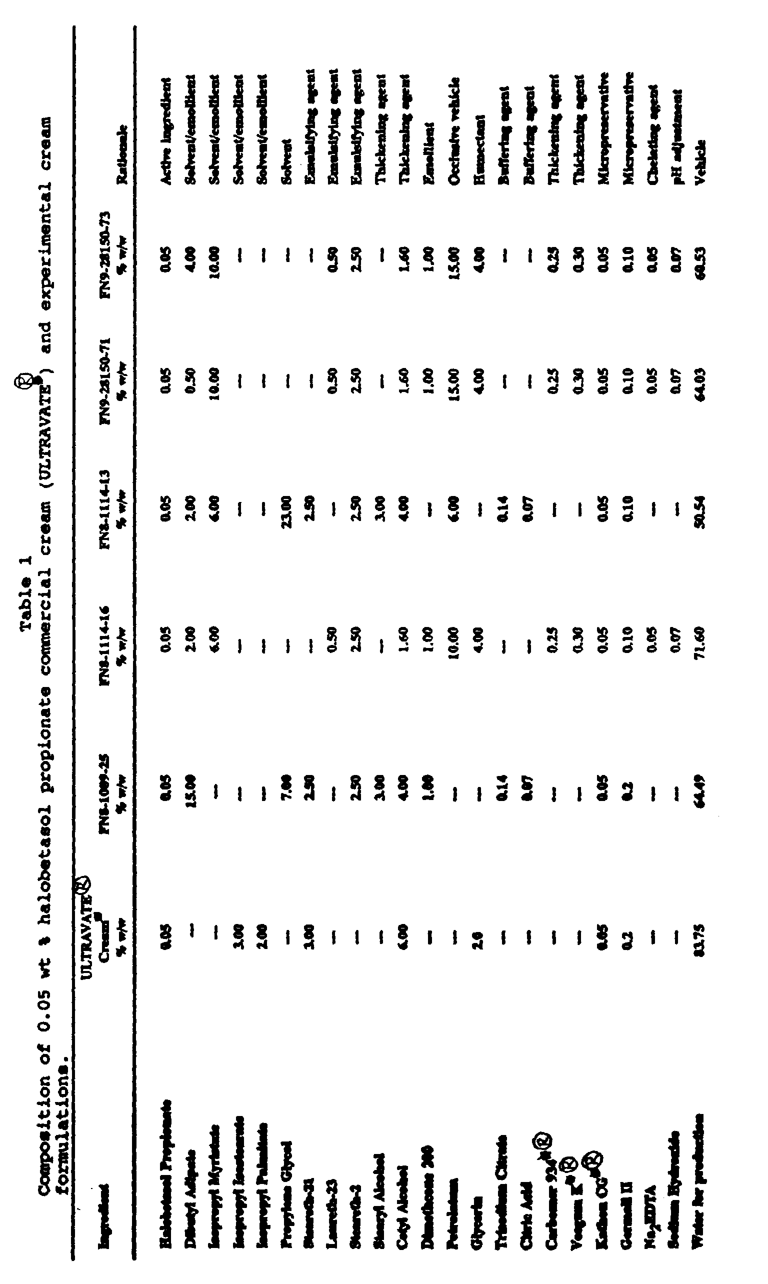

**Table 2**

| Composition of 0.05 wt % halobetasol propionate commercial ointment (ULTRAVATE®) and experimental ointment formulations. | | | | |
|---|---|---|---|---|
| Ingredients | ULTRAVATE® Ointment % w/w | FN9-28150-79 % w/w | FN9-28150-80 % w/w | Rationale |
| Halobetasol Propionate | 0.05 | 0.05 | 0.05 | Active Ingredient |
| Dibutyl Adipate | --- | 2.00 | 2.00 | Solvent/emollient |
| Isopropyl Myristate | --- | 6.00 | 6.00 | Solvent/emollient |
| Propylene Glycol | 7.50 | --- | --- | Solvent |
| Dehymuls E® | 7.50 | 7.50 | 7.50 | Emulsifying agent |
| Bees Wax | 5.00 | 7.50 | 2.50 | Thickening agent |
| Ozokerite | --- | --- | 5.00 | Occlusive vehicle |
| Petrolatum | 79.95 | 76.95 | 76.95 | Occlusive vehicle |

**Table 3**

| In-vitro skin penetration of 0.05 wt % halobetasol propionate from different formulations. | | |
|---|---|---|
| Formulation | Amount penetrated in 72 hours (micrograms)* | Amount penetrated in percentage relative to ULTRAVATE® Ointment as Control |
| Study I | | |
| ULTRAVATE® Ointment (PG 7 wt %) | 2.29 | 100.00 |
| ULTRAVATE® Cream (Isopropyl Isoslearate 3 wt % + Isopropyl palmitate 2 wt %) | 0.36 | 16.59 |
| FN8-1089-25 cream (DBA 15 wt % + PG 7 wt %) | 0.35 | 15.28 |

| Study II | | |
|---|---|---|
| ULTRAVATE® Ointment (PG 7 wt %) | 4.76 | 100.00 |
| FN8-1114-16 cream (DBA 2 wt % + IPM 6 wt %) | 3.9 | 81.90 |
| FN8-1114-13 cream (DBA 2 wt % + IPM 6 wt % + PG 23 wt %) | 2.15 | 45.16 |

| Study III | | |
|---|---|---|
| ULTRAVATE® Ointment (PG 7 wt %) | 3.75 | 100.00 |
| FN9-28150-71 cream (DBA 0.5 wt % + IPM 10 wt %) | 4.75 | 126.00 |
| FN9-28150-73 cream (DBA 4 wt % + IPM 10 wt %) | 2.61 | 69.6 |

| Study IV | | |
|---|---|---|
| ULTRAVATE® Ointment (PG 7 wt %) | 3.75 | 100.00 |
| FN9-28150-79 ointment (DBA 2 wt % + IPM 6 wt %) | 3.48 | 92.80 |
| FN9-28150-80 ointment (DBA 2 wt % + IPM 8 wt % + Ozokerite 5 wt %) | 5.57 | 148.50 |

| | | |
|---|---|---|
| * Amount in micrograms penetrated per cm² area of the skin. The results are average of multiple determinations. | | |

### EXAMPLE 3

### Rhino Mouse Utricle Model To Evaluate Efficacy Of Retinoid Formulations

The skin of rhino mouse is characterized by the presence of numerous large cysts resembling comedones (S. J. Mann, Hairloss and cyst formation in hairless and rhino mutant mice, Anat. Rec., Vol. 170, pages 485-500, 1971) and is useful as a model system for the pharmacologic testing of agents, such as retinoids, for the treatment of acne (R. E. Ashton, M. J. Connor and N. J. Lowe, Histological changes in the skin of the rhino mouse induced by retinoids, J. Invest. Derm., Vol. 82: pages 632-635, 1984; E. J. Van Scott, Experimental animal integumental models for screening potential dermatologic drugs, in: Advances in Biology of the Skin, Vol. XII. Pharmacology and the Skin, W. Motagna, E. J. Van Scott and R. B. Stoughton, Editors, Meredith Corporation, New York, pages 523-531, 1972.) The experimental details of the model used to evaluate the efficacy of tretinoin formulations are given below.

Female rhino mice were obtained from the Skin and Cancer Hospital, Temple University, Philadelphia, Pennsylvania. Upon receipt, the rhino mice were treated with an anti-parasitic agent, trichlorfon (COMBOT®), to rid them of pinworms. It was administered in their drinking water for two weeks. An additional two weeks was allowed to pass without COMBOT® treatment before mice were placed on study. Animals were housed in accordance with the National Institute of Health guidelines and had free access to food and water. At the time of the study initiation, the mice were 9-14 weeks old.

The mice were individually housed in a plastic shoe box cage with corn cob bedding and placed in rooms lighted with yellow lights, cycled for 12 hours on and 12 hours off during the study.

The mice were divided into groups of five and their contralateral flank sites were treated with 50 microliters test formulations and vehicle or ethanol. One group of mice was run as a control with treatment of vehicle or ethanol. Animals were treated once a day for five days.

Two days after the last treatment, the animals were sacrificed by CO₂ inhalation, and the skin sections from the test and control sites were taken. Excessive fat and connective tissue from the subcutaneous region of the skin were removed using a scalpel. The skin was then placed on filter paper and a biopsy of 7/8" of the circular area of skin was removed by arch punch. The epidermal sheet from the biopsy was separated from the dermis after incubation in 0.5% acetic acid solution for 10-20 hours at 4°C. These sheets were then fixed in formalin, dehydrated with ethanol and cleared in xylene.

The sheets were then evaluated for changes in the sizes of the utriculi as follows: The maximum diameter of 20 representative utriculi per tissue sample were measured, using an IBM PC computer image measurement program (Microscience, Inc., Federal Way, Washington D.C.) and an Olympus microscope (200x magnification).

Retinoid effects were evaluated as a percent reduction in utricle diameter by comparing mean diameter of the vehicle treated groups to that of formulation treated groups.

### EXAMPLE 4

Formulations containing 1% BMY 30047 (11-cis, 13-cis-12-hydroxymethyl retinoic acid, delta lactone) developed in a vehicle (30047-C-03-A) similar to the marketed RETIN-A® cream and in other experimental creams are shown in Table 4 and Table 5 respectively.

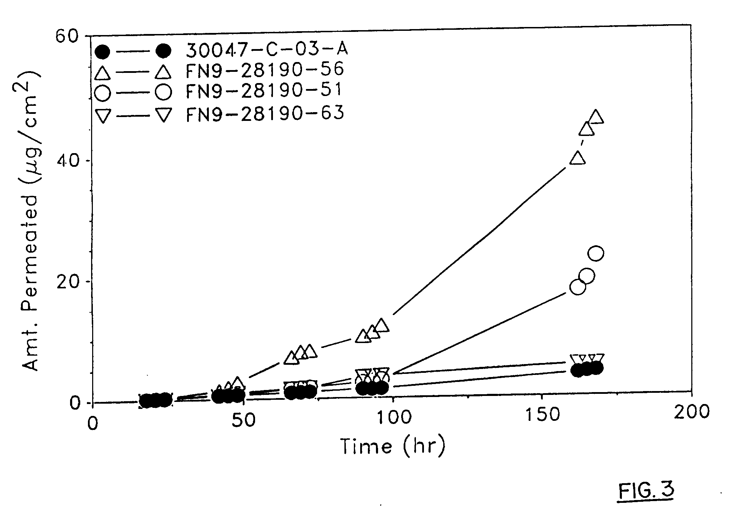

The in vitro human skin penetration of BMY 30047 from these formulations is shown in Fig. 3 and Table 6. The rank order of penetration is FN9-28140-56 cream ≥ FN9-28150-51 cream ≥, FN9-28150-63 cream ≥, 30047-C-03-A cream.

The FN9-28190-56 cream containing 20 wt % DBA and 10 wt % IPM gave as much as nine times the skin penetration of BMY 30047 than that of the cream having 15 wt % IPM and 0.1 wt % sodium lauryl sulfate (30047-C-03-A) and the cream containing 10 wt % IPM, 20 wt % Capmul MCM® and 0.1 wt % octoxynol-9 (FNS-28190-63).

The skin penetration of the cream containing 30 wt % DBA (FN9-28150-51) was about one-half the penetration of the cream containing 20 wt % DBA and 10 wt % IPM (FN9-28190-56) and about five times the penetration of the other two experimental creams.

It can be concluded from this study that dibutyl adipate enhances the skin penetration of BMY 30047 and that the skin penetration is further enhanced when isopropyl myristate is used in conjunction with dibutyl adipate. The data suggests that there is synergism in penetration enhancement when DBA and IPM are used together.

**Table 4**

| Composition 1 wt % BMY 30047 Cream (30047-C-03-A) | | |
|---|---|---|
| Ingredient | 30047-C-03-A % W/W | Rationale |
| BMY 30047 | 1.00 | Active Ingredient |
| Stearic Acid | 18.00 | Thickening Agent |
| Isopropyl Myristate | 15.00 | solvent/Emollient |
| PEG-40-Stearate | 5.00 | Emulsifying Agent |
| Stearyl Alcohol | 2.00 | Thickening Agent |
| Xanthan Gum | 0.60 | Thickening Agent |
| Sorbic Acid | 0.20 | Micropreservative |
| BHA | 0.10 | Antioxidant |
| BHT | 0.10 | Antioxidant |
| Sodium Lauryl Sulfate | 0.10 | Emulsifying Agent |
| Water for Production | 57.90 | vehicle |

**Table 5**

| Composition of 1 wt % BMY 30047 creams | | | | |
|---|---|---|---|---|
| Ingredient | FN9-28190-56 | FN9-28190-51 | FN9-28190-63 | Rationale |
| BMY 30047 | 1.00 | 1.00 | 1.00 | Active Ingredient |
| Dibutyl Adipate | 20.00 | 30.00 | --- | Solvent/Emollient |
| Isopropyl Myristate | 10.00 | --- | 10.00 | Solvent/Emollient |
| Capmul MCM® | --- | --- | 20.00 | Solvent/Emollient |
| Octoxynol-9 | --- | --- | 0.10 | Emulsifying Agent |
| Steareth-2 | 2.50 | 2.50 | 2.50 | Emulsifying Agent |
| Steareth-21 | 2.50 | 2.50 | 2.50 | Emulsifying Agent |
| Cetyl Alcohol | 2.00 | 2.00 | 2.00 | Thickening Agent |
| BHA | 0.075 | 0.075 | 0.075 | Antioxidant |
| BHT | 0.075 | 0.075 | 0.075 | Antioxidant |
| Stearyl Alcohol | 7.00 | 7.00 | 7.00 | Thickening Agent |
| Glyceryl Stearate | 1.00 | 1.00 | 1.00 | Emulsifying Agent |
| Laureth-4 | 1.00 | 1.00 | 1.00 | Emulsifying Agent |
| Glycerin | 2.00 | 2.00 | 2.00 | Humectant |
| Na₂EDTA | 0.05 | 0.05 | 0.05 | Chelating Agent |
| Methyl Paraben | 0.20 | 0.20 | 0.20 | Micropeservative |
| Propyl Paraben | 0.20 | 0.20 | 0.20 | Micropreservative |
| Germall II® | 0.20 | 0.20 | 0.20 | Micropreservative |
| Water for Production | 50.20 | 50.20 | 50.20 | Vehicle |

**Table 6**

| In-vitro skin penetration of 1.0 wt % BMY 30047 in different cream formulations. | |
|---|---|
| Formulation | Amount of BMY 30047 penetrated in 168 hours (micrograms/cm²)** |
| FN9-28190-56 (20 wt % DBA + 10 wt % IPM) | 45.66 |
| FN9-28190-51 (30 wt % DBA) | 23.15 |
| FN9-28190-63 (CAPMUL MCM* 20 wt % + IPM 10 wt % + Octoxynol-9 0.1 wt %) | 5.33 |
| 30047-C-03-A (IPM 15 wt % + 0.1 wt % SLS)*** | 4.20 |

| | |
|---|---|
| * Capmul MCM is glyceryl caprylate/caprate | |
| ** Average of 2 or 3 determinations | |
| *** SLS is sodium lauryl sulfate | |

### EXAMPLE 5

Tretinoin is a retinoid used topically for the treatment of acne, and more recently, A.M. Kligman (U.S. Patent 4,603,146), discloses the use of tretinoin in the treatment of photodamaged human facial skin. Tretinoin is marketed by Johnson & Johnson under the trade name RETIN-A®.

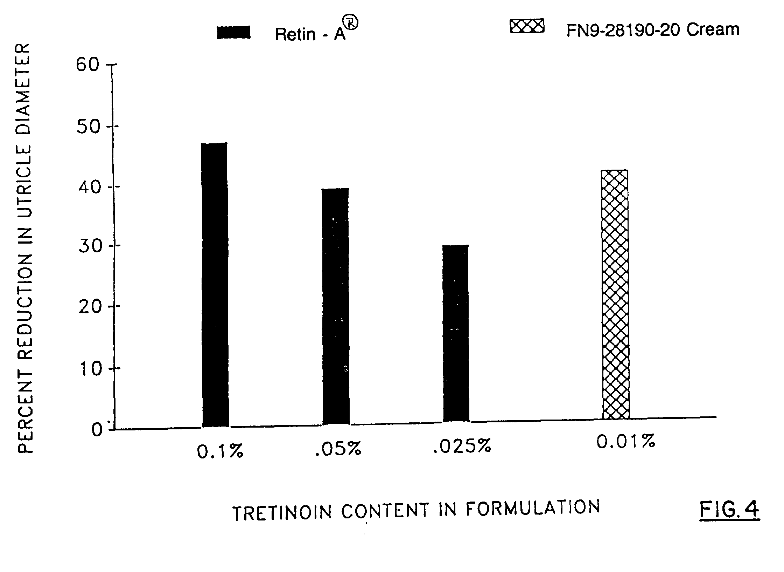

The composition of the experimental 0.01 wt % tretinoin cream formulation is given in Table 7. For the purpose of evaluating the efficacy of tretinoin in experimental formulation in comparison to the marketed RETIN-A® cream they were evaluated in the rhino mouse utricle model. The test results are shown in Figure 4. The rank order of efficacy is: RETIN-A® cream (0.1 wt %) > FN9-28190-20 cream (0.01 wt %) = RETIN-A® cream (0.05 wt %) > RETIN-A® cream 0.025 wt %.

The FN9-28190-20 cream contains 3 wt % DBA, in an amount just sufficient to solubilize the tretinoin in the formulation. It is clear from this example that the formulation containing DBA increases the efficacy and hence the topical bioavailability of tretinoin. The FN9-28190-20 cream contains a five-fold reduced amount of tretinoin in comparison to RETIN-A ®cream containing 0.05 wt % tretinoin while maintaining the same activity.

**Table 7**

| The composition of 0.01 wt % tretinoin cream | | |
|---|---|---|
| Ingredients | FN9-28190-20 % w/w | Rationale |
| Tretinoin | 0.01 | Active Ingredient |
| Dibutyl adipate | 3.00 | Solvent/Emollient |
| Mineral oil | 4.00 | Solvent/Emollient |
| Stearyl alcohol | 5.00 | Thickening Agent |
| Cetyl alcohol | 3.00 | Thickening Agent |
| Steareth-2 | 2.00 | Emulsifying Agent |
| Steareth 21 | 2.00 | Emulsifying Agnet |
| Glyceryl Stearate | 1.00 | Emulsifying Agent |
| Laureth-4 | 1.00 | Emulsifying Agent |
| BHA | 0.05 | Antioxidant |
| BHT | 0.05 | Antioxidant |
| Glycerin | 4.00 | Humectant |
| Citric acid | 0.004 | Chelating Agent |
| Kathon CG® | 0.05 | Micropreservative |
| Germall II® | 0.10 | Micropreservative |
| Water purified | 74.73 | Vehicle |

### EXAMPLE 6

Ammonium lactate is the ammonium salt of α-hydroxy acid (lactic acid). It is marketed by Westwood-Squibb under the trade name Lac-Hydrin® for the topical treatment of xerosis and ichthyosis. R. J. Yu et al, (U.S. Patent 4,105,783) and E. J. VanScott et al, (U.S. Patent 4,234,599) disclose the use of α- or β-hydroxy acid or α-keto acids and esters thereof, their amides and ammonium salts for therapeutic treatment of dry skin and skin keratoses.

The composition of experimental creams containing ammonium lactate equivalent to 30 wt % of lactic acid is given in Table 8. Three days prior to the in vitro skin penetration studies the experimental creams were homogeneously mixed with ¹⁴C-lactic acid (sodium salt) to result in experimental cream having radioactivity of about 0.01 microCi per mg.

The in vitro human skin penetration data of lactic acid from these formulations is shown in Table 9. The rank order of penetration is FN1-28393-46 > FN1-28393-45 > FN1-28393-47.

The FN1-28393-45 cream contains 5 wt % DBA. This cream showed about 1.4 times more penetration than FN1-28393-47 cream containing 5 wt % mineral oil.

The FN1-28393-46 cream contains 3 wt % DBA and 3 wt % IPM. This showed about 2.2 times more penetration than FN1-28393-47 cream containing 5% mineral oil.

It can be concluded from this study that dibutyl adipate enhances the skin penetration of lactic acid and that the skin penetration is further enhanced when isopropyl myristate is used in conjunction with dibutyl adipate.

**Table 8**

| Composition of experimental creams containing ammonium lactate equivalent to 30 wt % of lactic acid. | | | | |
|---|---|---|---|---|
| Ingredients | FN1-28393-47 % w/w | FN1-28393-45 % w/w | FN1-28393-46 % w/w | Rationale |
| Ammonium lactate | 53.00 | 53.00 | 53.00 | Active ingredient |
| Glyceryl stearate and PEG-100 stearate | 0.5 | 0.5 | 0.5 | Emulsifying agent |
| Mineral oil | 5.0 | --- | --- | Emollient |
| Dibutyl adipate | --- | 5.0 | 3.0 | Emollient |
| Isopropyl myristate | --- | --- | 3.0 | Emollient |
| Dimethicone 200 | 1.0 | 1.0 | 1.0 | Emollient |
| Steareth-2 | 2.5 | 2.5 | 2.5 | Emulsifying agent |
| Steareth-21 | 2.5 | 2.5 | 2.5 | Emulsifying agent |
| Stearyl alcohol | 3.5 | 3.5 | 3.5 | Thickening agent |
| Laureth-4 | 1.0 | 1.0 | 1.0 | Emulsifying agent |
| Cetyl alcohol | 0.5 | 0.5 | 0.5 | Thickening agent |
| Magnesium aluminum silicate | 2.0 | 2.0 | 2.0 | Thickening agent |
| Kathon CG® | 0.05 | 0.05 | 0.05 | Micropreservative |
| Germall II® | 0.2 | 0.2 | 0.2 | Micropreservative |
| Propylene glycol | 5.0 | 5.0 | 5.0 | Humectant |
| Water | 23.25 | 23.25 | 22.25 | Vehicle |

**Table 9**

| In-vitro human skin penetration of different cream formulations containing ammonium lactate equivalent to 30 wt % of lactic acid. | | |
|---|---|---|
| Formulation | Amount of C¹⁴ lactic acid penetrated in 72 hours (DPM/CM²)* | Amount penetrated in percentage relative to the formulation with mineral oil as a control |
| FN1-28393-47 (Mineral oil 5 wt %) | 13004 | 100.00 |
| FN1-28393-45 (DBA 5 wt %) | 18115 | 139.30 |
| FN1-28393-46 (DBA 3 wt % + IPM 3 wt %) | 28817 | 221.60 |

| | | |
|---|---|---|
| *The results are an average of multiple determination | | |

### EXAMPLE 7

### Twenty - One Day Cumulative Irritation Tests In Humans

This test was conducted to evaluate the irritation and allergic potential of formulations having DBA and IPM combination. This study method and modifications of it have been described in variety of publications. (A. M. Kligman and W. M. Wooding, A method for the measurement and evaluation of irritants on human skin, J. Invest. Dermatol., Vol. 49: pages 78-94, 1967; F. N. Marazulli and H. I. Maibach, in Dermatotoxicology, Ed. 2, Hemisphere Publishing Corporation, pages 167-296, 1983.)

The composition of test formulations BMY 30047 1 wt % cream (30047-C-09-A) and its vehicle 30047-C-10-A cream are given in Table 10. Both test formulations contain 20 wt % DBA and 10 wt % IPM. These test formulations and two control formulations (0.5 wt % sodium lauryl sulfate and RETIN-A® 0.1 wt % cream) were applied to 27 subjects under an occlusive patch for 21 consecutive days. Formulations were re-applied daily, with the exception that patches applied on Saturday were left undisturbed until Monday; the Monday evaluation also recorded for the previous Sunday. Skin reactions were scored on a 5-point grading scale as follows: 0 = no sign of irritation; 1 = slight erythema; 2 = noticeable erythema with slight infiltration; 3 = erythema with marked edema; 4 = erythema with edema and blistering. Once a score of 4 was observed at any site, no further applications were made, and a score of 4 was assigned for the duration of the study. Results were reported by summing scores from all subjects and all visits. Relative irritation potential was estimated based on comparison with scores of the control compounds.

The cumulative irritation potentials of the test formulations and the controls are given in Table 11. Both the test formulations having DBA and IPM were classified as causing "no significant irritation" as they had very low cumulative irritation score; whereas the control products RETIN-A® 0.1 wt % cream and 0.5 wt % sodium lauryl sulfate had a cumulative score of 432.4 and 285.6 respectively and were classified as "moderately irritating".

This study clearly demonstrates that this invention provides us with skin penetration enhancing agents with negligible side effects.

**Table 10**

| Composition of BMY 30047 cream and its vehicle | | |
|---|---|---|
| Ingredients | 30047-C-09-A % w/w | 30047-C-10-A % w/w |
| BMY 30047 | 1.0 | ---- |
| Dibutyl Adipate | 20.0 | 20.0 |
| Isopropyl Myristate | 10.0 | 10.0 |
| Steareth-2 | 2.5 | 2.5 |
| Steareth-21 | 2.5 | 2.5 |
| Stearyl Alcohol | 7.0 | 7.0 |
| Cetyl Alcohol | 2.0 | 2.0 |
| Glyceryl Stearate | 1.0 | 1.0 |
| Laureth-4 | 1.0 | 1.0 |
| BHA | 0.075 | 0.075 |
| BHT | 0.075 | 0.075 |
| Glycerin | 2.0 | 2.00 |
| Kathon CG® | 0.05 | 0.05 |
| Germall II® | 0.20 | 0.2 |
| Water for production | 50.60 | 51.60 |

**Table 11**

| Results of 21-day cumulative irritation test in humans. | |
|---|---|
| Formulation | Cumulative Irritation Score |
| 30047-C-09-A cream (1 wt % BMY 30047 + 20 wt % DBA + 10 wt % IPM) | 30.9 |
| 30047-C-10-A cream vehicle (20 wt % DBA + 10 wt % IPM) | 24.8 |
| Retin A® 0.1 wt % cream | 432.4 |
| 0.5 wt % sodium lauryl sulfate in petrolatum | 285.6 |

## Claims (Claims for the following Contracting State(s): AT, BE, CH, DE, DK, FR, GB, IT, LI, LU, MC, NL, PT, SE)

1. A topical composition for enhancing skin penetration of a pharmacologically active agent comprising:
a) an effective amount of a pharmacologically active agent selected from an α- or β-hydroxycarboxylic acid, ketocarboxylic acid or ester, lactone or salt thereof;
b) dibutyl adipate in an amount such that the pharmacologically active agent is present in the composition in the form of a suspension, saturated solution or solution having a degree of unsaturation of not more than 1.5; and
c) a pharmaceutically acceptable non-toxic topical vehicle.

2. A topical composition for enhancing skin penetration of a pharmacologically active agent comprising:
a) an effective amount of a pharmacologically active agent selected from a steroid, retinoid and arotinoid or from an α- or β-hydroxycarboxylic acid, ketocarboxylic acid or ester, lactone or salt thereof;
b) a mixture of dibutyl adipate and isopropyl myristate in an amount such that the pharmacologically active agent is present in the composition in the form of a suspension, saturated solution or solution having a degree of unsaturation of not more than 1.5; and
c) a pharmaceutically acceptable non-toxic topical vehicle.

3. The composition as claimed in claim 1 or 2, wherein the active agent is present in the form of a saturated solution.

4. The composition as claimed in claim 1 or 2, wherein the active agent is present in the form of a solution having a degree of unsaturation of not more than 1.5.

5. The composition as claimed in any one of the preceding claims, wherein the dibutyl adipate is present in an amount of from 0.1 wt % to 99 wt %, based on the weight of the composition, and the isopropyl myristate is present in an amount of 0 wt % to 50 wt %, based on the weight of the composition.

6. The composition as claimed in claim 5, wherein the dibutyl adipate is present in an amount of 0.1 wt % to 50 wt %, based on the weight of the composition, and the isopropyl myristate is present in an amount of 1 wt % to 30 wt %, based on the weight of the composition.

7. The composition as claimed in any one of the preceding claims, wherein the active agent is present in an amount of 0.001 wt % to 80 wt %, based on the weight of the composition.

8. The composition as claimed in any one of the preceding claims, wherein the active agent is selected from tretinoin, halobetasol propionate or 11-cis, 13-cis-12-hydroxymethyl retinoic acid δ-lactone or from an α- or β-Hydroxycarboxylic acid or salt thereof.

9. The composition as claimed in claim 8, wherein the α-hydroxycarboxylic acid or salt thereof is lactic acid or ammonium lactate.

10. The use of dibutyl adipate or a mixture of dibutyl adipate and isopropyl myristate together with a pharmacologically active agent for preparing a topical composition for controlled and enhanced dermal penetration as defined in any one of claims 1 to 9.

11. A process for preparing a topical composition as defined in any one of claims 1 to 9 which comprises incorporating the pharmacologically active agent into dibutyl adipate or a mixture of dibutyl adipate and isopropyl myristate and a pharmaceutically acceptable non-toxic topical vehicle.

## Claims (Claims for the following Contracting State(s): ES, GR)

1. A process for preparing a topical composition for enhancing skin penetration of a pharmacologically active agent comprising:
a) an effective amount of a pharmacologically active agent selected from an α- or β-hydroxycarboxylic acid, ketocarboxylic acid or ester, lactone or salt thereof;
b) dibutyl adipate in an amount such that the pharmacologically active agent is present in the composition in the form of a suspension, saturated solution or solution having a degree of unsaturation of not more than 1.5; and
c) a pharmaceutically acceptable non-toxic topical vehicle,
which comprises incorporating the pharmacologically active agent into dibutyl adipate and the pharmaceutically acceptable non-toxic topical vehicle.

2. A process for preparing a topical composition for enhancing skin penetration of a pharmacologically active agent comprising:
a) an effective amount of a pharmacologically active agent selected from a steroid, retinoid and arotinoid or from an α- or β-hydroxycarboxylic acid, ketocarboxylic acid or ester, lactone or salt thereof;
b) a mixture of dibutyl adipate and isopropyl myristate in an amount such that the pharmacologically active agent is present in the composition in the form of a suspension, saturated solution or solution having a degree of unsaturation of not more than 1.5; and
c) a pharmaceutically acceptable non-toxic topical vehicle,
which comprises incorporating the pharmacologically active agent into a mixture of dibutyl adipate and isopropyl myristate and the pharmaceutically acceptable non-toxic vehicle.

3. The process as claimed in claim 1 or 2, wherein the active agent is present in the form of a saturated solution.

4. The process as claimed in claim 1 or 2, wherein the active agent is present in the form of a solution having a degree of unsaturation of not more than 1.5.

5. The process as claimed in any one of the preceding claims, wherein the dibutyl adipate is present in an amount of from 0.1 wt % to 99 wt %, based on the weight of the composition, and the isopropyl myristate is present in an amount of 0 wt % to 50 wt %, based on the weight of the composition.

6. The process as claimed in claim 5, wherein the dibutyl adipate is present in an amount of 0.1 wt % to 50 wt %, based on the weight of the composition, and the isopropyl myristate is present in art amount of 1 wt % to 30 wt %, based on the weight of the composition.

7. The process as claimed in any one of the preceding claims, wherein the active agent is present in an amount of 0.001 wt % to 80 wt %, based on the weight of the composition.

8. The process as claimed in any one of the preceding claims, wherein the active agent is selected from tretinoin, halobetasol propionate or 11-cis, 13-cis-12-hydroxymethyl retinoic acid δ-lactone or from an α- or β-hydroxycarboxylic acid or salt thereof.

9. The process as claimed in claim 8 wherein the α-hydroxycarboxylic acid or salt thereof is lactic acid or ammonium lactate,

10. The use of dibutyl adipate or a mixture of dibutyl adipate and isopropyl myristate together with a pharmacologically active agent for preparing a topical composition for controlled and enhanced dermal penetration as defined in any one of claims 1 to 9.

## Patentansprüche (Patentansprüche für folgende(n) Vertragsstaat(en): AT, BE, CH, DE, DK, FR, GB, IT, LI, LU, MC, NL, PT, SE)

1. Topische Zusammensetzung zur Erhöhung der Hautpenetration eines pharmakologischen Wirkstoffs, umfassend:
a) eine wirksame Menge eines pharmakologischen Wirkstoffs, der ausgewählt ist unter einer α- oder β-Hydroxycarbonsäure, Ketocarbonsäure oder einem Ester, Lacton oder Salz davon;
b) Dibutyladipat in solch einer Menge, daß der pharmakologische Wirkstoff in der Zusammensetzung als Suspension, gesättigte Lösung oder Lösung mit einem Ungesättigtheitsgrad von höchstens 1,5 vorliegt; und
c) ein pharmazeutisch akzeptables, nichttoxisches, topisches Vehikel.

2. Topische Zusammensetzung zur Erhöhung der Hautpenetration eines pharmakologischen Wirkstoffs, umfassend:
a) eine wirksame Menge eines pharmakologischen Wirkstoffs, der ausgewählt ist unter einem Steroid, Retinoid und Arotinoid, oder einer α- oder β-Hydroxycarbonsäure, Ketocarbonsäure oder einem Ester, Lacton oder Salz davon;
b) ein Gemisch aus Dibutyladipat und Isopropylmyristat in solch einer Menge, daß der pharmakologische Wirkstoff in der Zusammensetzung als Suspension, gesättigte Lösung oder Lösung mit einem Ungesättigtheitsgrad von höchstens 1,5 vorliegt; und
c) ein pharmazeutisch akzeptables, nichttoxisches, topisches Vehikel.

3. Zusammensetzung nach Anspruch 1 oder 2, worin der Wirkstoff als gesättigte Lösung vorliegt.

4. Zusammensetzung nach Anspruch 1 oder 2, worin der Wirkstoff als Lösung mit einem Ungesättigtheitsgrad von höchstens 1,5 vorliegt.

5. Zusammensetzung nach einem der vorhergehenden Ansprüche, worin Dibutyladipat in einer Menge von 0,1 Gew.-% bis 99 Gew.-%, bezogen auf das Gewicht der Zusammensetzung, und Isopropylmyristat in einer Menge von 0 Gew.-% bis 50 Gew.-%, bezogen auf das Gewicht der Zusammensetzung, vorhanden ist.

6. Zusammensetzung nach Anspruch 5, worin Dibutyladipat in einer Menge von 0,1 Gew.-% bis 50 Gew.-%, bezogen auf das Gewicht der Zusammensetzung, und Isopropylmyristat in einer Menge von 1 Gew.-% bis 30 Gew.-%, bezogen auf das Gewicht der Zusammensetzung, vorhanden ist.

7. Zusammensetzung nach einem der vorhergehenden Ansprüche, worin der Wirkstoff in einer Menge von 0,001 Gew.-% bis 80 Gew.-%, bezogen auf das Gewicht der Zusammensetzung, vorhanden ist.

8. Zusammensetzung nach einem der vorhergehenden Ansprüche, worin der Wirkstoff ausgewählt ist unter Tretinoin, Halobetasolpropionat oder 11-cis-13-cis-12-Hydroxymethylretinsäure-δ-lacton oder einer α- oder β-Hydroxycarbonsäure oder einem Salz davon.

9. Zusammensetzung nach Anspruch 8, worin die α-Hydroxycarbonsäure oder ein Salz davon Milchsäure bzw. Ammoniumlactat ist.

10. Verwendung von Dibutyladipat oder eines Gemisches aus Dibutyladipat und Isopropylmyristat zusammen mit einem pharmakologischen Wirkstoff zur Herstellung einer wie in einem der Ansprüche 1 bis 9 definierten topischen Zusammensetzung zur kontrollierten und erhöhten dermalen Penetration.

11. Verfahren zur Herstellung einer wie in einem der Ansprüche 1 bis 9 definierten topischen Zusammensetzung, wobei man den pharmakologischen Wirkstoff in Dibutyladipat oder in ein Gemisch aus Dibutyladipat und Isopropylmyristat, und ein pharmazeutisch akzeptables, nichttoxisches, topisches Vehikel einbringt.

## Patentansprüche (Patentansprüche für folgende(n) Vertragsstaat(en): ES, GR)

1. Verfahren zur Herstellung einer topischen Zusammensetzung zur Erhöhung der Hautpenetration eines pharmakologischen Wirkstoffs, umfassend:
a) eine wirksame Menge eines pharmakologischen Wirkstoffs, der ausgewählt ist unter einer α- oder β-Hydroxycarbonsäure, Ketocarbonsäure oder einem Ester, Lacton oder Salz davon;
b) Dibutyladipat in solch einer Menge, daß der pharmakologische Wirkstoff in der Zusammensetzung als Suspension, gesättigte Lösung oder Lösung mit einem Ungesättigtheitsgrad von höchstens 1,5 vorliegt; und
c) ein pharmazeutisch akzeptables, nichttoxisches, topisches Vehikel,
wobei man den pharmakologischen Wirkstoff in Dibutyladipat und das pharmazeutisch akzeptable, nicht toxische topische Vehikel einbringt.

2. Verfahren zur Herstellung einer topischen Zusammensetzung zur Erhöhung der Hautpenetration eines pharmakologischen Wirkstoffs, umfassend:
a) eine wirksame Menge eines pharmakologischen Wirkstoffs, der ausgewählt ist unter einem Steroid, Retinoid und Arotinoid, oder einer α- oder β-Hydroxycarbonsäure, Ketocarbonsäure oder einem Ester, Lacton oder Salz davon;
b) ein Gemisch aus Dibutyladipat und Isopropylmyristat in solch einer Menge, daß der pharmakologische Wirkstoff in der Zusammensetzung als Suspension, gesättigte Lösung oder Lösung mit einem Ungesättigtheitsgrad von höchstens 1,5 vorliegt; und
c) ein pharmazeutisch akzeptables, nichttoxisches, topisches Vehikel,
wobei man den pharmakologischen Wirkstoff in ein Gemisch aus Dibutyladipat und Isopropylmyristat und das pharmazeutisch akzeptable, nichttoxische Vehikel einbringt.

3. Verfahren nach Anspruch 1 oder 2, wobei der Wirkstoff als gesättigte Lösung vorliegt.

4. Verfahren nach Anspruch 1 oder 2, wobei der Wirkstoff als Lösung mit einem Ungesättigtheitsgrad von höchstens 1,5 vorliegt.

5. Verfahren nach einem der vorhergehenden Ansprüche, wobei Dibutyladipat in einer Menge von 0,1 Gew.-% bis 99 Gew.-%, bezogen auf das Gewicht der Zusammensetzung, und Isopropylmyristat in einer Menge von 0 Gew.-% bis 50 Gew.-%, bezogen auf das Gewicht der Zusammensetzung, vorhanden ist.

6. Verfahren nach Anspruch 5, wobei Dibutyladipat in einer Menge von 0,1 Gew.-% bis 50 Gew.-%, bezogen auf das Gewicht der Zusammensetzung, und Isopropylmyristat in einer Menge von 1 Gew.-% bis 30 Gew.-%, bezogen auf das Gewicht der Zusammensetzung, vorhanden ist.

7. Verfahren nach einem der vorhergehenden Ansprüche, wobei der Wirkstoff in einer Menge von 0,001 Gew.-% bis 80 Gew.-%, bezogen auf das Gewicht der Zusammensetzung, vorhanden ist.

8. Verfahren nach einem der vorhergehenden Ansprüche, wobei der Wirkstoff ausgewählt ist unter Tretinoin, Halobetasolpropionat oder 11-cis-13-cis-12-Hydroxymethylretinsäure-δ-lacton oder einer α- oder β-Hydroxycarbonsäure oder einem Salz davon.

9. Verfahren nach Anspruch 8, wobei die α-Hydroxycarbonsäure oder ein Salz davon Milchsäure bzw. Ammoniumlactat ist.

10. Verwendung von Dibutyladipat oder eines Gemisches aus Dibutyladipat und Isopropylmyristat zusammen mit einem pharmakologischen Wirkstoff zur Herstellung einer wie in einem der Ansprüche 1 bis 9 definierten topischen Zusammensetzung zur kontrollierten und erhöhten dermalen Penetration.

## Revendications (Revendications pour l'(les) Etat(s) contractant(s) suivant(s): AT, BE, CH, DE, DK, FR, GB, IT, LI, LU, MC, NL, PT, SE :)

1. Composition topique pour améliorer la pénétration dans la peau d'un agent pharmacologiquement actif comprenant :
a) une quantité efficace d'un agent pharmacologiquement actif sélectionné parmi un acide α- ou β- hydroxycarboxylique, un acide cétocarboxylique ou son ester, la lactone ou son sel ;
b) du dibutyl adipate en une quantité telle que l'agent phramacologiquement actif soit présent dans la composition sous la forme d'une suspension, d'une solution saturée ou d'une solution ayant un degré d'insaturation de pas plus de 1,5; et
c) un véhicule topique non toxique pharmaceutiquement acceptable.

2. Composition topique pour améliorer la pénétration dans la peau d'un agent pharmacologiquement actif comprenant :
a) une quantité efficace d'un agent pharmacologiquement actif sélectionné parmi un stéroïde, un rétinoïde et un arotinoïde ou un acide α- ou β-hydroxycarboxylique, un acide cétocarboxylique ou un ester, une lactone ou son sel ;
b) un mélange de dibutyl adipate et d'isopropyl myristate en une quantité telle que l'agent pharmacologiquement actif soit présent dans la composition sous la forme d'une suspension, d'une solution saturée ou d'une solution ayant un degré d'insaturation de pas plus de 1,5; et
c) un véhicule topique non toxique pharmaceutiquement acceptable.

3. Composition selon la revendication 1 ou 2 où l'agent actif est présent sous la forme d'une solution saturée.

4. Composition selon la revendication 1 ou 2 où l'agent actif est présent sous la forme d'une solution ayant un degré d'insaturation de pas plus de 1,5.

5. Composition selon l'une quelconque des revendications précédentes, où le dibutyl adipate est présent en une quantité de 0,1 % an poids à 99% an poids an se basant sur le poids de la composition et l'isopropyl myristate est présent en une quantité de 0% en poids à 50% en poids an se basant sur le poids de la composition.

6. Composition selon la revendication 5 où le dibutyl adipate est présent en une quantité de 0,1% en poids à 50% en poids, an se basant sur le poids de la composition et l'isopropyl myristate est présent an une quantité de 1% en poids à 30% en poids, en se basant sur le poids de la composition.

7. Composition selon l'une quelconque des revendications précédentes, où l'agent actif est présent en une quantité de 0,001 % an poids à 80%, an poids an se basant sur le poids de la composition.

8. Composition selon l'une quelconque des revendications précédentes où l'agent actif est sélectionné parmi la trétinoïne, le propionate de l'halobétasol, ou la δ-lactone de l'acide 11-cis, 13-cis-12-hydroxyméthyl rétinoïque, ou parmi un acide α-ou β-hydroxycarboxylique ou son sel.

9. Composition selon la revendication 8 où l'acide α-hydroxycarboxylique ou son sel est l'acide lactique ou le lactate d'ammonium.

10. Utilisation de dibutyl adipate ou d'un mélange de dibutyl adipate et d'isopropyl myristate avec un agent pharmacologiquement actif pour la préparation d'une composition topique pour une pénétration dermique contrôlée et améliorée telle que définie selon l'une des revendications 1 à 9.

11. Procédé de préparation d'une composition topique telle que définie selon l'une des revendications 1 à 9 qui comprend l'incorporation de l'agent pharmacologiquement actif dans du dibutyl adipate ou un mélange de dibutyl adipate et d'isopropyl myristate et un véhicule topique non toxique pharmaceutiquement acceptable.

## Revendications (Revendications pour l'(les) Etat(s) contractant(s) suivant(s): ES, GR)

1. Procédé pour la préparation d'une composition topique pour améliorer la pénétration dans la peau d'un agent pharmacologiquement actif comprenant :
a) une quantité efficace d'un agent pharmacologiquement actif sélectionné parmi un acide α- ou β- hydroxycarboxylique, un acide cétocarboxylique ou son ester, la lactone ou son sel ;
b) du dibutyl adipate en une quantité telle que l'agent phramacologiquement actif soit présent dans la composition sous la forme d'une suspension, d'une solution saturée ou d'une solution ayant un degré d'insaturation de pas plus de 1,5 ; et
c) un véhicule topique non toxique pharmaceutiquement acceptable,
qui comprend l'incorporation de l'agent pharmacologiquement actif dans du dibutyl adipate et le véhicule topique non toxique pharmaceutiquement acceptable.

2. Procédé de préparation d'une composition topique pour améliorer la pénétration dans la peau d'un agent pharmacologiquement actif comprenant :
a) une quantité efficace d'un agent pharmacologiquement actif sélectionné parmi un stéroïde, un rétinoïde et un arotinoïde ou un acide α- ou β-hydroxycarboxylique, un acide cétocarboxylique ou ester, une lactone ou son sel ;
b) un mélange de dibutyl adipate et d'isopropyl myristate en une quantité telle que l'agent pharmacologiquement actif soit présent dans la composition sous la forme d'une suspension, d'une solution saturée ou d'une solution ayant un degré d'insaturation de pas plus de 1,5 ; et
c) un véhicule topique non toxique pharmaceutiquement acceptable,
qui comprend l'incorporation de l'agent pharmacologiquement actif dans un mélange de dibutyl adipate et d'isopropyl myristate et le véhicule non toxique pharmaceutiquement acceptable.

3. Procédé de la revendication 1 ou 2 où l'agent actif est présent sous la forme d'une solution saturée.

4. Procédé de la revendication 1 ou 2 où l'agent actif est présent sous la forme d'une solution ayant un degré d'insaturation de pas plus de 1,5.

5. Procédé de l'une quelconque des revendications précédentes, où le dibutyl adipate est présent en une quantité de 0,1% en poids à 99% en poids, en se basant sur le poids de la composition et l'isopropyl myristete est présent en une quantité de 0% en poids à 50% en poids, en se basant sur le poids de la composition.

6. Procédé de la revendication 5 où le dibutyl adipate est présent en une quantité de 0,1% en poids à 50% en poids, en se basant sur le poids de la composition et l'isopropyl myristate est présent en une quantité de 1% en poids à 30% en poids en se basant sur le poids de la composition.

7. Procédé de l'une quelconque des revendications précédentes, où l'agent actif est présent en une quantité de 0,001 % en poids à 80% en poids, en se basant sur le poids de la composition.

8. Procédé de l'une quelconque des revendications précédentes où l'agent actif est sélectionné parmi la trétinoïne, le propionate de l'halobétasol, ou la δ-lactone de l'acide 11-cis, 13-cis-12-hydroxyméthyl rétinoïque, ou parmi un acide α-ou β-hydroxycarboxylique ou son sel.

9. Procédé selon la revendication 8 où l'acide α-hydroxycarboxylique ou son sel est l'acide lactique ou le lactate d'ammonium.

10. Utilisation de dibutyl adipate ou d'un mélange de dibutyl adipate et d'isopropyl myristate avec un agent pharmacologiquement actif pour la préparation d'une composition topique pour une pénétration dermique contrôlée et améliorée telle que définie selon l'une des revendications 1 à 9.
